# EUROPEAN PATENT SPECIFICATION

(11) **EP 3 766 466 B1**
(45) Date of publication and mention of the grant of the patent: **21.02.2024**
(21) Application number: 19768092.9
(22) Date of filing: 11.03.2019
(51) Int. Cl.: A61F 13/475, A61F 13/533, A61F 13/472, A61F 13/536

(54) **ABSORBENT ARTICLE**
ABSORBIERENDER ARTIKEL
ARTICLE ABSORBANT

(30) Priority: 16.03.2018 JP 2018048764
(43) Date of publication of application: 20.01.2021
(73) Proprietor: DAIO PAPER CORPORATION, Shikokuchuo-shi Ehime 799-0492 (JP)
(72) Inventor: TAGOMORI, Junta, Sakura-shi, Tochigi 329-1411 (JP)
(74) Representative: Epping - Hermann - Fischer
(86) International application number: PCT/JP2019/009743
(87) International publication number: WO 2019/176865

(56) References cited:
- EP-A1- 3 677 234
- WO-A1-2012/014957
- WO-A1-2013/047715
- WO-A1-2015/076189
- WO-A1-2016/088400
- WO-A1-2017/038030
- JP-A- 2009 082 480
- JP-A- 2011 130 962

## Description

### Technical Field

The present invention primarily relates to absorbent articles used in sanitary napkin.

### Background Art

Conventionally, the absorbent article is known to include an absorbent article including a cotton pulp or the like between an impermeable backside sheet, such as a polyethylene sheet or a polyethylene sheet laminate nonwoven fabric, and a permeable surface sheet, such as a nonwoven fabric or a permeable plastic sheet.

In order to ensure the prevention of leakage of body fluid, it is effective to use a body fluid absorber having an excellent function to absorb and retain the body fluid. However, in order to maximize the absorption capacity of the body fluid provided by the absorber, it is particularly important to expand the diffusion region of the body fluid over a wide area of the absorbent article without any unevenness of the diffusion region of the body fluid in a specific part of the absorber.

Body fluid absorbed into the absorber enters the voids between the fibers, and diffuses between the fibers by capillary action. In this case, the body fluid that diffuses between fibers has the property of easily diffusing between fibers from large areas to small areas. That is, it is known that by forming the compressed portion of the absorber, the body fluid easily diffuses toward the compressed portion.

As an absorbent article forming such a compressed portion, for example, Patent Document 1 discloses an absorbent article with a pair of rear compressed portions separated from each other by at least compressing an absorbent core and a centerline passing through a center in a width direction, wherein the pair of rear compressed portions are formed along a front-back direction in the rear side region, wherein the pair of rear compressed portions are positioned behind the position where the hip flap becomes maximum in the width direction, and where the pair of rear compressed portions have a wide portion that increases the length in the width direction toward the rear side.

In addition, in the paragraph 0031 of Patent Document 2, an absorbent article with a front groove formed from a central portion having a forward convex shape and a pair of sides running in the central portion extending toward the rear is disclosed.

### Related Art Documents

### Patent Document

Patent Document 1: Japanese Unexamined Patent Application Publication No. 2017-328
Patent Document 2: Japanese Unexamined Patent Publication No. 2007-275491

Further, document WO 2013/047715 A1 relates to absorbent articles such as sanitary napkins, panty liners, incontinence pads and the like for absorbing menstrual blood and vaginal discharge, and more specifically, a concave portion formed of a combination of a wavy pattern and a dot pattern on a liquid-permeable surface sheet It is related with the absorbent article which formed.

Document WO 2015/076189 A1 relates to absorbent articles such as sanitary napkins and incontinence pads.

Document WO 2012/014957 A1 relates to an absorbent article that uses a porous plastic film imparted with a fiber touch feeling as a surface sheet.

### Summary of the Invention

### Problem to be Solved by the Invention

In the absorbent article described in Patent Document 1, a rear compressed portion inclined outward in the width direction with increasing distance from the front is provided. Because the rear compressed portion is provided separately from each other by a centerline passing through the center in the width direction of the absorbent article, the body fluid easily diffuses backward from the rear compressed portion through the gap between the pair of rear compressed portions, which may cause leakage from the end of the absorbent article.

On the other hand, in the absorbent article described in Patent Document 2, because the center in the width direction of the front groove projects forward and formed in a continuous linear shape substantially positioned on the longitudinal centerline of the absorbent article, the body fluid diffusing along the front groove is concentrated in the middle portion in the width direction of the front groove. As a result, the body fluid overflows at the pointed tip portion of the front groove and easily diffuses to the front side of the front groove, which may cause leakage from the end of the absorbent article.

Therefore, the main object of the present invention is to provide an absorbent article that prevents leakage from the end of the absorber by forming a compressed portion in a pattern that makes it easier to change the direction of diffusion of the body fluid that diffuses from the region corresponding to the body fluid discharging portion.

### Means to Solve the Problem

The invention is defined by the claims.
To solve the above-mentioned problem, according to an embodiment of the present invention, there is provided an sanitary napkin including a top sheet, a back sheet, and an absorbent body disposed between the top sheet and the back sheet, including:
a leakproof compressed portion constituted of a depressed portion formed by depressing at least the absorbent body, configured to prevent a body fluid from leaking an end of the absorbent body by changing a diffusion direction of the body fluid diffusing from a region corresponding to a body fluid discharging portion of a wearer, and formed in a predetermined pattern outside the body fluid discharging portion corresponding region,
wherein the leakproof compressed portion includes a liquid induction portion that is arranged on both sides of a centerline directed from a proximal side to a distal side, that includes a portions whose separation distance on both sides of the centerline decreases toward the distal side from the proximal side, and that is configured to introduce the body fluid diffusing outward from the body fluid discharging portion corresponding region, a body fluid collection portion located on the centerline and more distant from the body fluid discharging portion corresponding region than the liquid induction portion and configured to collect the body fluid introduced by the liquid induction portion, and a liquid diffusion portion configured to diffuse the collected liquid onto both sides of the centerline,
wherein a separation distance between the leakproof compressed portion and an edge of the absorbent body is not less than 10 mm,
wherein the liquid induction portions on both sides of the centerline has an approximately inverted V shape without its bonded apex as seen from the proximal side to the distal side,
wherein a separation width between ends of the liquid induction portions on the centerline side is from 2 mm to 10 mm, and
wherein a length of the portions whose separation distance on both sides of the centerline decreases toward the distal side from the proximal side is not less than 1/2 relative to the entire length of the liquid induction portion in a direction perpendicular to the centerline.

### Advantage of the Invention

As described in detail above, according to the present invention, by forming the compressed portion in a pattern that makes it easy to change the direction of diffusion of the body fluid diffusing from the region corresponding to the body fluid discharging portion, leakage from the end of the absorbent article can be prevented.

### Brief Description of the Drawings

FIG. 1 is a partially broken development view of a sanitary napkin 1 according to an embodiment;
FIG. 2 is a cross-sectional view taken along a line II-II of FIG. 1;
FIG. 3 is a cross-sectional view taken along a line III-III of FIG. 1;
FIG. 4 is a plan view of a leakproof compressed portion 11;
FIG. 5 is a plan view of a liquid induction portion according to a modification example;
FIG. 6 is a plan view illustrating a high compressed part 13a of a liquid collection portion;
FIG. 7 is a plan view of a leakproof compressed portion 11 according to a modification example;
FIG. 8 is a plan view of a leakproof compressed portion 11 according to a modification example;
FIG. 9 is a plan view of a sanitary napkin 1 according to a modification example;
FIG. 10 is a plan view (No. 1) of a sanitary napkin 1 according to another embodiment;
FIG. 11 is a plan view (No. 2) of a sanitary napkin 1 according to another embodiment; and
FIG. 12 is a plan view (No. 3) of a sanitary napkin 1 according to another embodiment.

### Mode for Carrying Out the Invention

In the following, embodiments are described below with reference to the accompanying drawings.

### [Basic structure of sanitary napkin 1]

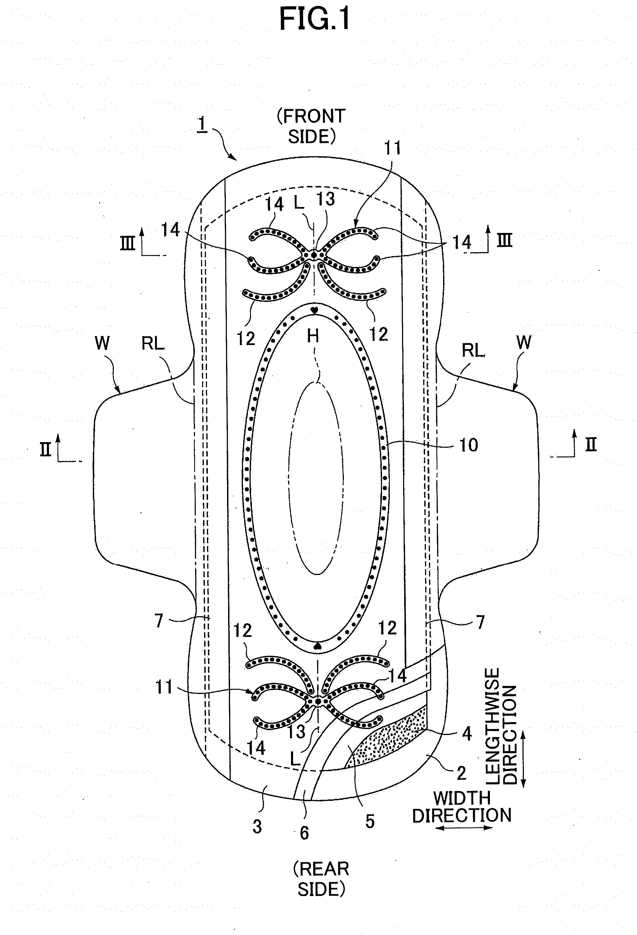

As shown in Figs. 1 to 3, the sanitary napkin 1 according to an embodiment includes a liquid impermeable back sheet 2 made of a polyethylene sheet and the like, a liquid permeable top sheet 3 that quickly passes menstrual blood and a vaginal discharge (that are collectively referred to as a body fluid hereinafter), an absorbent body 4 made of cotton pulp or synthetic pulp or the like interposed between the both sheets 2 and 3, and side non-woven fabrics 7 and 7 formed at both edge portions of the skin contact surface side throughout the approximately entire length in a lengthwise direction. Then, around the upper and lower edges of the absorbent body 4, the back sheet 2 and the top sheet 3 are bonded to each other by bonding means such as an adhesive such as a hot melt, a heat seal, an ultrasonic seal, or the like, and the back sheet 2 and the side non-woven fabric 7 extending laterally from the absorbent body 4 are bonded to each other by bonding means such as an adhesive such as a hot melt, a heat seal, an ultrasonic seal, or the like, so that a pair of right and left wing flaps W and W are formed in the area corresponding to the body fluid discharging portion of the wearer (hereinafter, referred to as the "the body fluid discharging portion corresponding region H"). In the illustrated example, the absorbent body 4 is surrounded by an encapsulant sheet 5 made of crepe paper or non-woven fabric for maintaining the shape and improving the diffusivity of the absorbent body 4, but the encapsulant sheet 5 is not required. In the illustrated example, a second sheet 6, which is made of a non-woven, hydrophilic material having a substantially similar shape to the surface sheet 3, is disposed adjacent to the non-skin side of the top sheet 3. However, the second sheet 6 is not required.

The body fluid discharging portion corresponding region H is a region disposed facing the body fluid discharging portion (including a vaginal opening) of the wearer when the sanitary napkin 1 is worn. The range in the napkin lengthwise direction of the region H corresponding to the body fluid discharge portion may be made the range of the napkin lengthwise direction at the proximal ends of a wing-shaped flap W when the wing-shaped flap W is formed on both sides, as in the case of the present sanitary napkin 1. When the wing-shaped flap W is not formed, the range of the napkin lengthwise direction may be made the range of the length of 70 to 90 mm in the napkin lengthwise direction from the position 60 to 80 mm behind the front edge of the sanitary napkin 1. The width of the napkin may be within the spaced width of the compressed portions 10 and 10 for the body fluid discharging portion when the compressed portions 10 and 10 for the body fluid discharging portion are formed on both sides of the area corresponding to the body fluid discharging portion H, as in the case of the present sanitary napkin 1. When the compressed portions 10 for the body fluid discharging portion are not formed, the width may be within the range of 30 to 50 mm centering the longitudinal centerline of the sanitary napkin 1.

The structure of the sanitary napkin 1 will be further described below. As the back sheet 2, a sheet material having at least water blocking properties, such as polyethylene, is used, and it is preferable that the sheet has moisture permeability from the viewpoint of preventing evaporation. As the water blocking and moisture permeable sheet material, a microporous sheet is preferably used, which is obtained by melting and kneading an inorganic filler into an olefinic resin such as polyethylene or polypropylene to form a sheet, and then stretching the sheet in the uniaxial or biaxial direction. One or more adhesive layers (not shown) are formed along the lengthwise direction of the napkin on the non-skin side (outer surface) of the back sheet 2, and the sanitary napkin 1 may be fixed to the underwear when it is attached to the body. The back sheet 2 may be a polylaminated non-woven fabric made of a plastic film and a non-woven fabric.

Next, as the top sheet 3, a perforated or non-porous non-woven fabric or a porous plastic sheet is preferably used. As the material fibers constituting the non-woven fabric, in addition to synthetic fibers including olefinic type such as polyethylene or polypropylene, polyester type, and polyamide type and the like, regenerated fibers such as rayon and cupro, natural fibers such as cotton and the like, may be used. The non-woven fabric obtained by an appropriate processing method such as a thermal bond method, a span lace method, a spunbond method, a meltblown method, a needle punch method or the like may be used. Of these processing methods, an air-through-type thermal bond non-woven fabric having a large void between fibers (a non-woven fabric formed from heat-bonded fibers by a hot-air processor) is particularly preferred in the present sanitary napkin 1. When a large number of holes is formed in the top sheet 3, the body fluid is rapidly absorbed, and the top sheet 3 has excellent dry touch properties. The fibers of the non-woven fabric may be either long fibers or short fibers, preferably using short fibers to create a texture like a towel. Also, in order to facilitate an embossing process, it is preferable to use olefin fibers such as polyethylene or polypropylene having a relatively low melting point. Alternatively, core fibers having a high melting point and a sheath made of fibers having a low melting point may be preferably used as a core, a core-sheath type fiber, a side-by-side type fiber, or a split-type fiber composite fiber.

The absorbent body 4 interposed between the back sheet 2 and the top sheet 3 may be composed of, for example, pulp and superabsorbent polymer. The superabsorbent polymer can be incorporated into the pulp constituting the absorbent article, for example, as particulate powder. The pulp may be made of cellulosic fibers such as chemical pulp, dissolved pulp or the like obtained from wood, or artificial cellulosic fibers such as rayon, acetate or the like, and a coniferous pulp having longer fibers than a hardwood pulp is preferably used in terms of function and price.

Preferably, the present sanitary napkin 1 is a thin-type slim napkin having a low visibility of the absorbent body 4. When the surface of the absorbent body 4 is lowered, the absorption capacity per unit area of the absorber 4 is reduced, and the body fluid easily diffuses in the plane direction. Therefore, in the slim napkin, the effect of the present invention of preventing leakage from the end of the absorbent body 4 can be fully exerted by changing the direction of diffusion of the body fluid. The basis weight of the pulp may be 50 to 300 g/m², preferably 80 to 220 g/m², and the basis weight of the superabsorbent polymer may be 30 to 180 g/m², preferably 50 to 160 g/m². The texture of these pulps and superabsorbent polymers need not be constant and may vary with the location of the absorbent article. For example, a high absorbency portion can be formed from pulp and superabsorbent polymer whose unit weight is made high in the area corresponding to the wearer's body fluid drain H.

A synthetic fiber may be mixed with the absorbent body 4. The synthetic fiber may be a polyolefin such as polyethylene or polypropylene, a polyester such as polyethylene terephthalate or polybutylene terephthalate, a polyamide such as nylon, and a copolymer thereof, or may be a mixture of these two types. Alternatively, core fibers with high melting point and low melting point fiber sheaths may be used as core sheath fibers, side-by-side fibers, split fibers, and other composite fibers. In the case of hydrophobic fibers, it is preferable to use the synthetic fibers whose surface is treated with a hydrophilic agent so as to have an affinity for body fluids.

If necessary, the hydrophilic second sheet 6 disposed adjacent to the non-skin side of the top sheet 3 may have hydrophilic properties with respect to a body fluid. Specifically, a material having hydrophilicity itself may be used including regenerated fibers such as rayon or cupra, or natural fibers such as cotton. Otherwise, a fiber obtained by treating a surface of a synthetic fiber such as olefinic, polyester, or polyamide, such as polyethylene or polypropylene with a hydrophilic agent to provide hydrophilicity, may be used. In addition, a complex fiber such as a core-sheath type fiber including a core fiber having a high melting point and a sheath of fibers having a low melting point, a side-by-side type fiber, a split-type fiber or the like may be used. The second sheet 6 and the absorbent body 4 (the encapsulant sheet 5) are preferably bonded by hot melt adhesive or the like. By joining the second sheet 6 and the absorber 4, the body fluid can be quickly transferred from the second sheet 6 to the absorbent body 4.

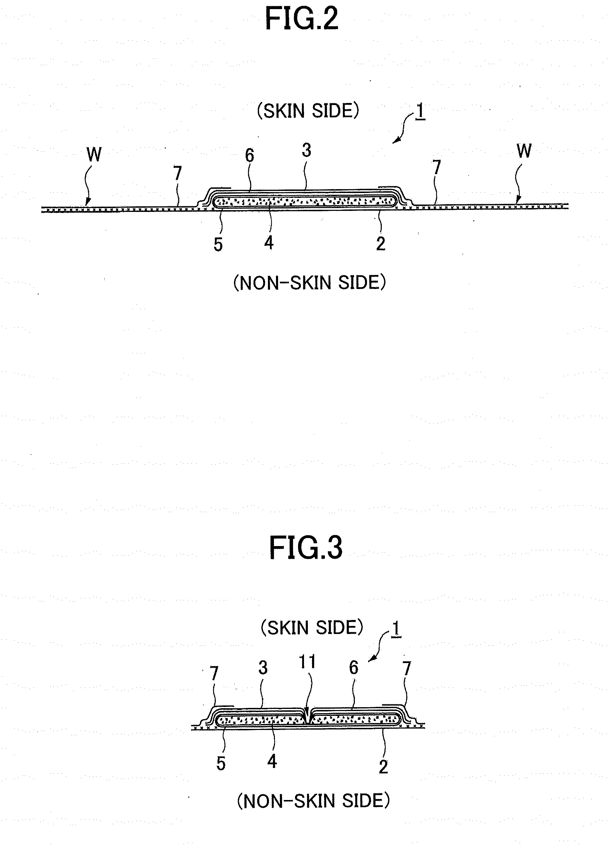

The width dimension of the top sheet 3 is, in the illustrated example, slightly longer than the width of the absorbent body 4 as shown in the cross-sectional views in Figs. 2 and 3, only covering the absorbent body 4. A side non-woven fabric 7 that is different from the top sheet 3 is provided outside the top sheet 3, and the side non-woven fabric 7 is configured by using a non-woven fabric material that is appropriately treated with water-repellent treatment or hydrophilic treatment depending on the purpose of preventing the menstrual blood or the virginal discharge from permeating the top sheet 3 or increasing the feel for the skin.

The side non-woven fabric 7 may be made of natural fibers, synthetic fibers, or regenerated fibers by any suitable processing method. As the side non-woven fabric, it is preferable to use a non-woven fabric having an increased basis weight in order to increase the concealment of the body fluid. Specifically, it is desirable to use a non-woven fabric made of a weight of 13 to 23 g/m². In addition, a water-repellent non-woven fabric coated with silicon-based, paraffin-based, alkyl-chromic-based water repellent, or the like is preferably used to ensure prevention of permeation of body fluid.

As shown in Figs. 2 and 3, the side non-woven fabric 7 is bonded with an adhesive such as hot melt over a range from a predetermined internal position to an outer edge of the back sheet 2, and a flap portion in which the absorbent body 4 is not interposed is formed on both sides of the absorbent body 4 by the laminated sheet portion of the side non-woven fabric 7 and the back sheet 2. The flap portion allows a pair of right and left wing flaps W and W to be formed longitudinally at a position on the side of the absorber corresponding to the body fluid discharging portion corresponding region H. An adhesive layer (not shown) is provided on the outer surfaces of the wing-shaped flaps W and W, respectively, so that the wing-shaped flaps W and W are folded back to the opposite side at the folding line RL position at the proximal end when mounted to the shorts, and are wound around the crotch portion of the shorts and secured thereto. Meanwhile, the inner side portion of the side non-woven fabric 7 is bonded to the absorbent body 4 side (the skin side of the top sheet 3) while being laminated to the skin side of the top sheet 3.

### <Compressed Portion>

In the present sanitary napkin 1, at least a compressed portion formed by depressing the absorbent body 4 is formed in a predetermined pattern in a predetermined area. The compressed portion may be formed by depressing only the absorbent body 4 by compressing the absorbent body 4 from the skin side surface (the surface of the top sheet 3 side), or may integrally depress the encapsulant sheet 5 and the absorbent body 4 by compressing the encapsulant sheet 5 from the skin side surface of the encapsulant sheet 5 covering the skin side surface of the absorbent body 4, or may depress the top sheet 3 integrally from the surface sheet 3 to the absorbent body 4 by compressing the top sheet 3 from the skin side surface while laminating the surface sheet 3 and the second sheet 6 as necessary on the skin side of the absorbent body 4. Among them, in addition to the diffusion of the body fluid absorbed by the absorber 4, in order to reduce the diffusion of the body fluid absorbed by the top sheet 3 and to prevent leakage of the body fluid flowing through the surface of the top sheet 3, the aforementioned compression portion is preferably formed by the depression portion integrally depressed from the top sheet 3 to the absorbent body 4.

In the sanitary napkin 1 shown in Fig. 1, as the aforementioned pressure portion, the compressed portion 10 for the body fluid discharging portion disposed along a lengthwise direction of the napkin on at least both sides of the body fluid discharging portion corresponding to the body fluid discharging portion H and blocking the discharged body fluid, and a pressure portion 11 for a leakproof purpose disposed outside the body fluid discharging portion corresponding to the body fluid discharging portion and preventing leakage from the end portion of the absorbent body 4 by changing the diffusion direction of the body fluid diffused from the body fluid discharging portion corresponding region H corresponding to the body fluid discharging portion.

The compressed portion for body fluid discharging portion 10 is provided to express resistant force against leg pressure or the like caused by the inner portion of the base of the leg acting in the width direction inward from both sides when being worn, to fit the area surrounded by the compressed portions for the body fluid discharging portion 10 and 10 to the body fluid discharging portion of the wearer, to block the body fluid absorbed by the absorbent body 4 or the body fluid flowing through the surface of the top sheet 3, and to prevent the body fluid from diffusing outward from the compressed portion for the body fluid discharging portion 10.

As the compressed portion for body fluid discharging portion 10, a known form disposed at least on both sides of the body fluid discharging portion corresponding region H can be widely used. For example, in the illustrated example, it is formed by an elliptical shape surrounding the body fluid discharging portion corresponding region H. Alternatively, it may be formed by a pair of arcuate lines separately disposed on both sides of the body fluid discharging corresponding region H, or it may be formed by a pattern with an approximately U-shape (approximately C-shape) in which the rear end or the front end is opened, that is, a symmetrical pattern with a convex or concave curve-shaped in the width direction outward. The compressed portion for body fluid discharging portion 10 is made of a continuous line pressed over the entire length in the illustrative example, but may be formed of an intermittent line in which the non-compressed portion is intermittently provided.

The compressed portion for body fluid discharging portion 10 may be configured by a compressed groove having a substantially uniform depth, but as shown in the drawings, it is preferable that the compressed portion 10 be composed of a low compressed portion and a high compressed portion having a greater depth than the low compressed portion. In the drawing, the black filled portion is the high compressed portion and the other portion is the low compressed portion. A plurality of the aforementioned high compressed portions is arranged at predetermined intervals. As the shape of the high compressed portion in a plan view, a well-known shape can be used, for example, a circular, semi-circular, elliptical, heart-shaped, star-shaped, or a combination of two or more of these.

Next, the above-mentioned leakproof compressed portion 11 will be described. The sanitary napkin 1 is characterized in that this leakproof compressed portion 11 is provided. The leakproof compressed portion 11 is used to prevent leakage from the end of the absorbent body 4 by changing the direction of diffusion of the body fluid that diffuses inside the absorbent body 4 from the body fluid discharging portion corresponding region H.

The leakproof compressed portion 11 is provided outside the body fluid discharging portion corresponding region H, and preferably outside the body fluid discharging portion corresponding region H spaced apart from the body fluid discharging portion 10. Accordingly, the body fluid that diffuses outward beyond the compressed portion for body fluid discharging portion 10 can be prevented from diffusing toward the end of the absorbent body 4, and the leakage prevention effect from the end of the absorbent body 4 can be improved. The distance (minimum separation length in a line) from the compressed portion for body fluid discharging portion 10 is not particularly limited, but it may be 5 to 30 mm, preferably 7 to 15 mm, because it is likely to flow continuously from the compressed portion for body fluid discharging portion 10 when the distance is too short and it is difficult to aggregate when the distance is too long due to the expansion of the diffusion area of the body fluid. On the other hand, the separation distance (the minimum distance in a straight line) between the leakproof compressed portion 11 and the edge of the absorbent body 4 is not particularly limited. The separation distance be not less than 10 mm in order to prevent leakage from the edge of the absorbent body 4.

Similar to the above-described compressed portion for body fluid discharging portion 10, the foregoing leakproof compressed portion 11 may be configured by a compressed groove having a substantially uniform groove depth. However, as illustrated in the drawings, the foregoing leakproof compressed portion 11 preferably comprises a low compressed portion and a high compressed portion having a greater groove depth than the low compressed portion.

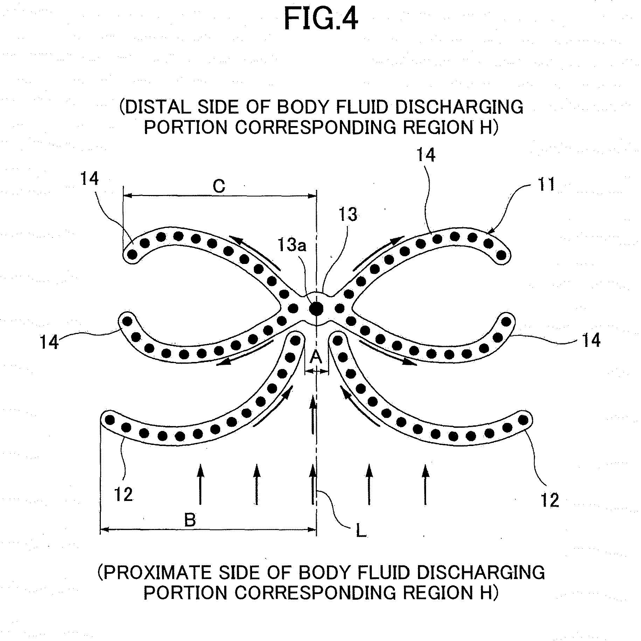

As a pattern in a plan view of the leakproof compressed portion 11, as shown in FIG. 4 in detail, includes a liquid induction portion 12 disposed on both sides of a centerline L from a proximal side to a distal side of a fluid discharging portion corresponding region H, and has portions arranged on both sides of the centerline L and whose distance decreases with increasing distance from the proximal side, a collection portion 13 located on the centerline L and on the distal side of the liquid induction portion 12, and a liquid diffusion portion 14 extending from the collection portion 13 to both sides of the centerline L, thereby forming a generally ribbon shape (bowknot shape).

The liquid induction portion 12 has an action of directing a body fluid diffusing outward from the body fluid discharging portion corresponding region H to the L side of the central line. The body fluid diffusing from the body fluid discharging portion corresponding region H first reaches the liquid induction portion 12 among the leakproof compressed portion 11 and is guided along the liquid induction portion 12 toward the centerline L.

As shown in Fig. 4, the liquid induction portions 12 and 12 are arranged on both side of the centerline L, and preferably they are separated from each other by the centerline L between the liquid inductions 12 and 12. As shown in Fig. 4, when the proximal side of the fluid discharging portion corresponding region H is positioned downward and the distal side is positioned upward, the liquid induction portions 12 and 12 on both sides are arranged in a substantially mountain shape without its apex (generally inverted V-shaped without the apex bonded). By positioning both sides of the centerline L apart from each other, the body fluid diffusing outward from the body fluid discharging portion corresponding region H is likely to flow through the separation portion provided in the central portion. The separation width A is from 2 mm to 10 mm, preferably from 2 mm to 7 mm. When it is less than 2 mm, the body fluid is unlikely to pass through the space between the liquid induction portions 12 and 12 on both sides, and when it is larger than 10 mm, unless the collection portion 13 is formed in a large shape, the body fluid cannot be received by the collection portion 13, and the body fluid easily flows outside the leakproof compressed portion 11.

It is preferable that the liquid induction portion 12 be formed by a curve that expands to the side of the body fluid discharging portion corresponding region H in terms of facilitating the diffusion of the body fluid toward the centerline L, but this is not limited thereto. The liquid induction portion 12 may be formed by a curve or a straight line that expands to the distal side of the body fluid discharging portion corresponding region H.

The liquid induction portion 12 may have portions that decreases the distance from each other crossing the centerline L with increasing distance from the proximal side of the body fluid discharging portion corresponding region H at least on the side of the centerline L. The portion outside the above-mentioned portion may be formed so that the tip of the distal side (the tip on the distal side in the width direction) of the body fluid discharging portion corresponding region H is curved as shown in the drawings. The separation distance of the portions (the length in the direction perpendicular to the centerline L) that decreases the distance with increasing distance from the proximal side, may be set to any distance. However, in order to obtain the effect of sufficiently guiding the diffusing body fluid toward the centerline L along the liquid induction portion 12, the length is not less than 1/2, preferably not less than 2/3, relative to the entire length of the liquid induction portion 12 (the length in the direction perpendicular to the centerline L). In addition, it is preferable that the length B from the centerline L to the tip of the liquid induction portion 12 be longer than the length C from the centerline L to the tip of the liquid diffusion portion 14 so that the body fluid diffusing from the proximal side to the distal side of the body fluid discharging portion corresponding end H does not directly reach the liquid diffusion portion 14 (B>C).

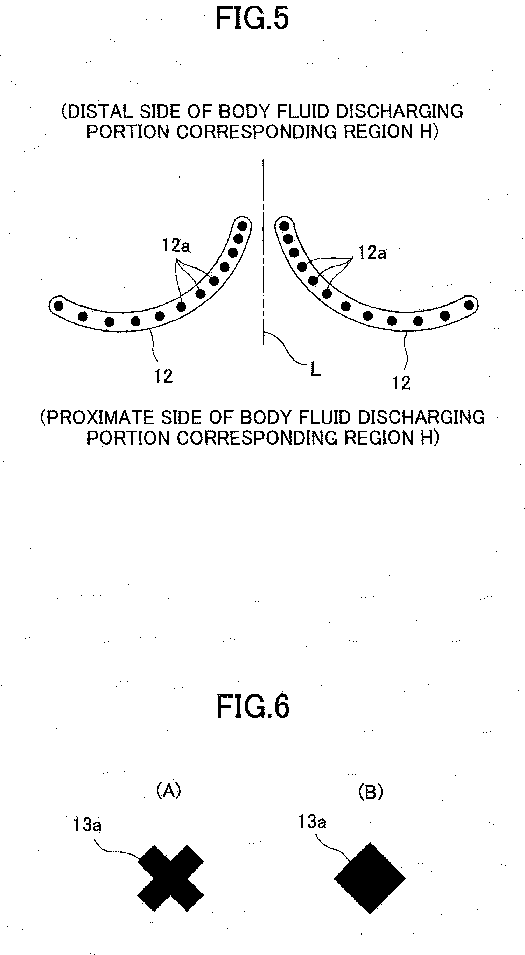

Preferably, the liquid induction portion 12 is formed so as to gradually increase the average density of the fibers toward the centerline L. Accordingly, in the liquid induction portion 12, the body fluid easily diffuses toward the centerline L due to the capillary action of the fiber, and the body fluid is easily concentrated in the collection portion 13. The average density of fibers is an average value of density of fibers per unit area. In order to gradually increase the average density of fibers, because the average density of fibers can be increased by narrowing the space of the high compressed portion 12a having a relatively high fiber density, the liquid induction portion 12 may be formed of a low compressed portion and a high compressed portion 12a as shown in FIG. 5, and the space of the high compressed portion 12a may be gradually narrowed toward the centerline L.

The collection portion 13 has an action of receiving and collecting body fluid guided to the centerline L side by the liquid induction portions 12 and 12 on both sides. The collection portion 13 serves as a starting point for diffusing the liquid to the subsequent liquid diffusion portion 14.

The collection portion 13 is positioned on the centerline L and on the distal side, more distant from the body fluid discharging portion corresponding region H than the centerline L-side tips of the liquid induction portion 12. That is, the collection portion 13 is positioned adjacent to the tips of the liquid induction portions 12 and 12 so as to block the gap between the liquid induction portions 12 and 12 disposed on both sides of the centerline L. Therefore, a body fluid diffusing outward through a spaced gap between the liquid induction portions 12 and 12 is prevented from diffusing outward by the collection portion 13.

The collection portion 13 may be positioned consecutively at the end of the liquid induction portion 12 on the centerline L side, or may be positioned spaced apart from the end of the liquid induction portion 12 as shown in FIG. 4. When the liquid is disposed continuously, the body fluid is easily transferred from the end of the liquid induction portion 12 to the collection portion 13, and the subsequent diffusion to the liquid diffusion portion 14 is smoothly performed. On the other hand, when the fluid induction portion 12 and the collection portion 13 are disposed apart, because there is a relatively large air gap between the fibers of the non-compressed portion, the transfer of the body fluid from the liquid induction portion 12 to the collection portion 13 is slowed, and the body fluid is prevented from rapidly concentrating in the collection portion 13, overflowing in the collection portion 13 and diffusing outward, thereby preventing leakage. The separation distance (minimum separation length in a straight line) between the collection portion 13 and the liquid induction portion 12 is not particularly limited, but preferably is about 0 to 5 mm.

The shape in a plan view of the collection portion 13 is preferably, but not particularly limited, substantially circular in order to receive body fluid and to disperse the collected body fluid. In the example shown in Fig. 4, a single high compressed portion 13a is disposed in the center of a generally circular low compressed portion. Thus, the direction of the diffusion of the body fluid is not limited, and the body fluid is dispersed almost evenly in any direction, thereby facilitating the diffusion of the bodily fluid.

The shape in a plan view of the high compressed portion 13a disposed in the collection portion 13 is not particularly limited, but it is preferable that the high compressed portion 13a be formed in a shape that is likely to cause liquid diffusion along the liquid diffusion portion 14. Specifically, in addition to the circular shape shown in Fig. 4, the diamond shape may be formed as an X-shape or a diamond shape (in the example shown, a square shape) as shown in Fig. 6. If it is circular, it is likely to diffuse approximately evenly in all directions. When the X-shape is used, the diffusion of the body fluid in the direction along the centerline L is reduced, and the diffusion is easily made in the oblique direction. In the case of a square, such as a diamond, it is easier to direct the diffusion of body fluid in the oblique direction (toward the apex) in the shape of the high compressed portion 13a. It is preferable that the diamond shape is a square shape arranged so that one side thereof is inclined at an angle of 45 degrees so as to prevent the diffusion of the body fluid along the centerline L (from the proximal side to the distal side of the region corresponding to the body fluid discharging portion H) and to facilitate the diffusion of the body fluid toward both sides of the centerline L, that is, so that any diagonal of the diagonal shape is arranged along the centerline L.

The high compressed portion 13a disposed in the collection portion 13 may be formed in an area substantially equivalent to the high compressed portion disposed in other portions, but is preferably formed in an area larger than the high compressed portion disposed in other portions. Thus, the function as a barrier of the collection portion 13 can be strengthened, and it is possible to prevent the transfer of body fluid through the collection portion 13 from the proximal side to the distal side of the body fluid discharging portion corresponding region H.

The liquid diffusion portion 14 has an action of allowing body fluid collected in the collection portion 13 to diffuse toward both sides of the centerline L. Thus, the direction of the body fluid diffusing outward from the body fluid discharging portion corresponding region H (the direction of the body fluid diffusing outward along the aforementioned centerline L) can be dispersed in both sides of the centerline L, thereby preventing the diffusion of the body fluid toward the end of the absorbent body 4 and preventing leakage of the body fluid.

The liquid diffusion portion 14 preferably extends continuously from the liquid collection portion 13 for rapidly diffusing the body fluid collected in the liquid collection portion 13. When the liquid diffusion portion 14 is connected to the liquid collection portion 13, the low compressed portions are connected to each other. In addition, when the liquid diffusion portion 14 is disposed on both sides of the centerline L as described below, if at least two liquid diffusion portions 14 and 14 are disposed discontinuously, the liquid diffusion portions 14 may be disposed separated from each other in a discontinuous manner.

The direction in which the liquid diffusion portion 14 extends may not be parallel to the direction in which the centerline L extends. In order to prevent the body fluid from diffusing directly from the liquid induction portion 12 to the liquid diffusion portion 14 proximate to the liquid induction portion 12 while preventing the body fluid from diffusing toward the end of the absorbent body 4, it is preferable that the direction in which the liquid diffusion portion 14 extends has a predetermined angle with respect to the centerline L. Specifically, the centerline L may be 30 degrees to 150 degrees, preferably 60 degrees to 120 degrees.

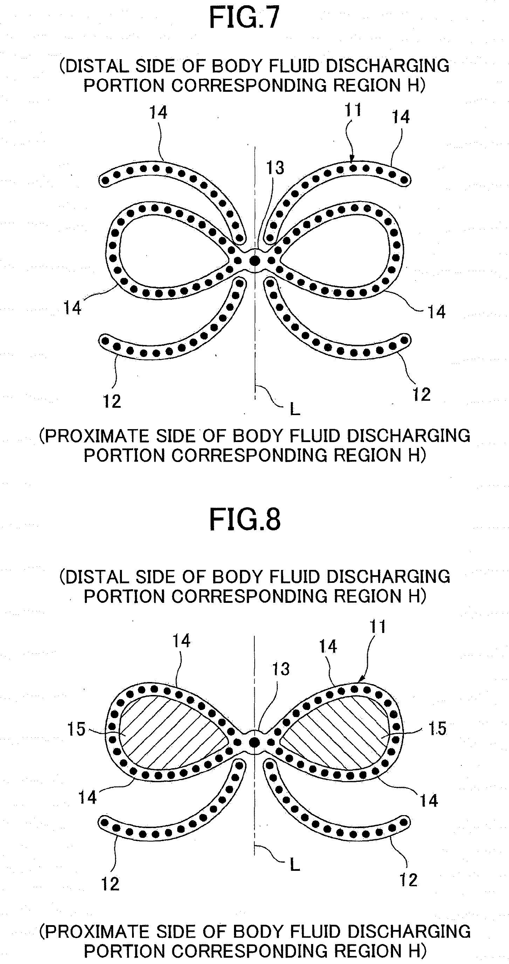

The liquid diffusion portion 14 may be arranged so as to extend as only a single portion from the liquid collection portion 13 to both sides of the centerline L, but it is preferable that the liquid diffusion portion 14 extends a plurality of times in a different direction. Thus, the body fluid collected in the collection portion 13 can quickly diffuse to both sides along the liquid diffusion portion 14, and it is possible to prevent the body fluid from overflowing the collection portion 13 and diffusing toward the end of the absorbent body 4. The number of the liquid diffusion portions 14 may be two as shown in Fig. 4, three as shown in Fig. 7, or four or more although it is not shown.

The shape in a plan view of the liquid diffusion portion 14 may be any shape, such as a straight line, a curve, a folded line, a wavy line, or the like. However, as shown in Figs. 4 and 7, the curved shape is preferable so that the body fluid easily diffuses along the liquid diffusion portion 14, and at the same time, when the liquid diffusion portions 14 and 14 are disposed on both sides of the centerline L, the area surrounded by the liquid diffusion portion 14 increases, so that more body fluid can be retained in this area. In particular, as shown in Figs. 4 and 7, when a plurality of liquid diffusing portions 14, ... are disposed on both sides of a centerline L, it is preferable to form at least the liquid diffusion portion 14 disposed on the proximal side of the body fluid discharging portion corresponding region H by a curve that expands to the distal side of the body fluid discharging portion corresponding region H, and at least the liquid diffusion portion 14 disposed on the distal side of the body fluid discharging portion corresponding region H by a curve that expands to the distal side of the body fluid discharging portion corresponding region H. This increases an area of the region surrounded by the liquid diffusion portions 14, 14 and allows more body fluid to be retained in this area.

When a plurality of liquid diffusion portions 14, ... are disposed on both sides of the aforementioned centerline L, as shown in Figs. 4 and 7, the tips (the outer portions in the width direction) of the liquid diffusion portions 14 and 14 disposed at the most proximal side and the distal side, respectively, of the body fluid discharging portion corresponding region H are preferably formed so as to gradually narrow the separation distances of each other with increasing distance from the liquid collection portion 13. Accordingly, the body fluid diffused to the tip of the liquid diffusion portion 14 tends to be retained in a region surrounded by the liquid diffusion portion 14, ... and thus it is possible to prevent the liquid from diffusing outward. As a result, the leak prevention effect from the end of the absorbent body 4 can be increased.

As shown in Figs. 7 and 8, at least two of the plurality of liquid diffusion portions 14, ... extending to both sides of the centerline L may be connected (coupled) at the tips. By connecting the tips with each other, the two liquid diffusion portions 14, 14 are formed in a closed loop shape, making it easier to retain body fluid in the loop.

In order to facilitate the retention of the body fluid in the region surrounded by the plurality of liquid diffusing portions 14, ... , the whole or a part of the region surrounded by the of liquid diffusing portions 14, ..., all of the region (the diagonal region in the figure) in the illustrated example, may include compressed regions 15, as shown in Fig. 8. The compressed region 15 is a depressed region formed with a depth approximately equal to or shallower than that of the low compressed portion of the liquid diffusion portion 14, wherein at least the absorbent body 4, preferably the top sheet 3 and the absorbent body 4, are integrally depressed from the surface of the top sheet 3. Thus, the inter-fiber space of the compressed region 15 becomes smaller than the inter-fiber space of the region outside the liquid diffusion portion 14, and the body fluid easily accumulates in the compressed region 15 due to the capillary action.

In the example shown in Fig. 1, a pattern in a plan view is formed substantially symmetrically with respect to the centerline L, and the centerline L is provided at a position substantially coincident with a longitudinal centerline passing through the width-direction-center portion of the sanitary napkin 1 on the front side and the rear side of the body fluid discharging corresponding region H, respectively. Thus, it is possible to prevent so-called front leakage and rear leakage, in which the body fluid diffusing forward and backward from the the body fluid discharging portion corresponding region H leaks from the front and rear ends of the absorbent body 4. When the pattern in a plan view of the foregoing leakproof compressed portion 11 is formed symmetrically with respect to the centerline L, the effect is noticeable in a position where the body position of the wearer is almost symmetrical, such as standing, sitting, or supine position.

The leakproof compressed portion 11 is provided only on one side of the front side and the rear side of the body fluid discharging portion corresponding region H, but not on the other side. The leakproof compressed portion 11 may be arranged in series or in parallel in each region. In addition, it may be disposed on both sides or in the diagonal direction of the body fluid discharging corresponding region H.

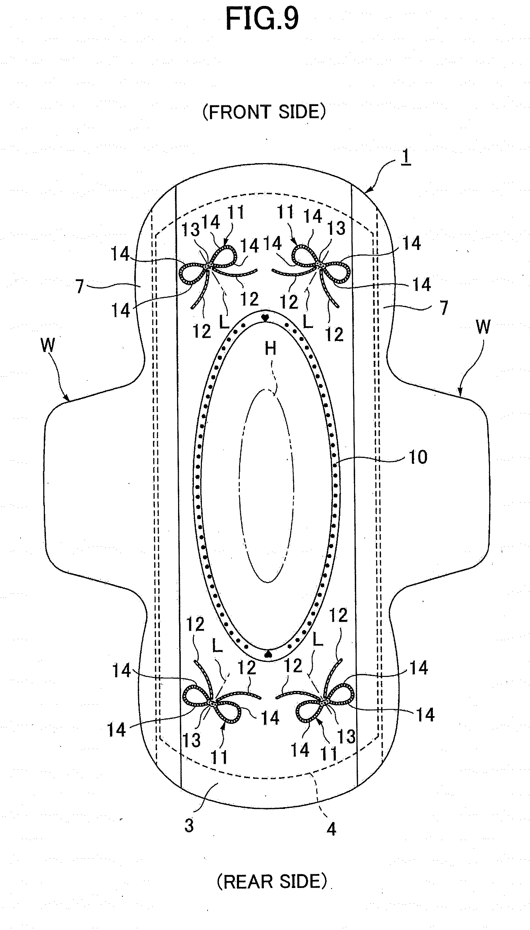

Meanwhile, the leakproof compressed portion 11 may be formed so that the pattern in a plan view is asymmetric to the centerline L as shown in Fig. 9. In this case, it is preferable that the arrangement of the leakproof compressed portion 11 be disposed at the diagonal position of the body fluid discharging portion corresponding region H (a position where the centerline L of the leakproof compressed portion 11 does not overlap the longitudinal center line extending longitudinally in the width direction of the central portion of the sanitary napkin 1, and is inclined at a predetermined angle to the longitudinal center line), as shown in the drawing example. Thus, the portion disposed on the side of the sanitary napkin 1 from the centerline L can have different functions on both sides of the centerline L, such that the portion disposed on the lateral side of the sanitary napkin 1 from the centerline L is formed in a pattern suitable for capturing body fluid diffusing in the width direction of the sanitary napkin 1 from the body fluid discharging portion corresponding region H, and the portion disposed on the central side of the sanitary napkin 1 from the centerline L is formed in a pattern suitable for capturing body fluid diffusing in the longitudinal direction of the sanitary napkin 1 from the body fluid discharging portion corresponding region corresponding region H. For example, in the example shown in Fig. 9, the separation distance between the liquid induction portion 12 and the liquid diffusion portion 14 is increased in the liquid diffusion portion 14 from the centerline L in the lateral portion of the sanitary napkin 1 to the central portion thereof so as to prevent the body fluid passing through the liquid induction portion 12 from moving to the liquid diffusion portion 14. Thus, because the body fluid flowing laterally to the body fluid discharging portion corresponding region H is reliably collected in the collection portion 13 by the liquid induction portion 12, and then dispersed in the plurality of liquid diffusion portions 14, ..., the amount of the body fluid flowing laterally to the absorbent body 4 can be reliably reduced. When formed in a laterally asymmetric pattern, the ability to inhibit fluid diffusion can be enhanced when the wearer is more likely to diffuse laterally in a particular position, such as in a lateral decubitus position. Although not shown, the amount of the body fluid diffusing to the edge of the absorbent body 4 may be further reduced by increasing the relative number of the diffusion portions 14 disposed at the central side of the sanitary napkin 1 from the centerline L.

In the sanitary napkin 1 having the above-described configuration, in the leakproof compressed portion 11, the body fluid that diffuses outward from the body fluid discharging portion corresponding region H is collected in the fluid collection portion 13 by the liquid induction portion 12, and then caused to diffuse toward both sides of the centerline L along the liquid diffusion portion 14, .... Accordingly, by providing the leakproof compressed portion 11, because the direction of the diffusion of the body fluid diffused from the body fluid discharging portion corresponding region H can be controlled in a direction different from the direction (the direction in which the centerline L extends) toward the end of the absorbent body 4, the leakage from the end of the absorbent body 4 can be prevented.

Further, as shown in Fig. 4, even when the liquid induction portion 12 cannot introduce the body fluid to the collection portion 13, and when the body fluid diffuses across the liquid induction portion 12, a plurality of liquid diffusion portions 14, ... are disposed at the distal side of the body fluid discharging portion corresponding region H. Therefore, the liquid diffusion portions 14, ... act as a levee to block the diffusion of the body fluid, and it is possible to prevent the body fluid from diffusing from the leakproof compressed portion 11 to the distal side of the body fluid discharging portion corresponding region H.

### [Examples of Other Embodiments]

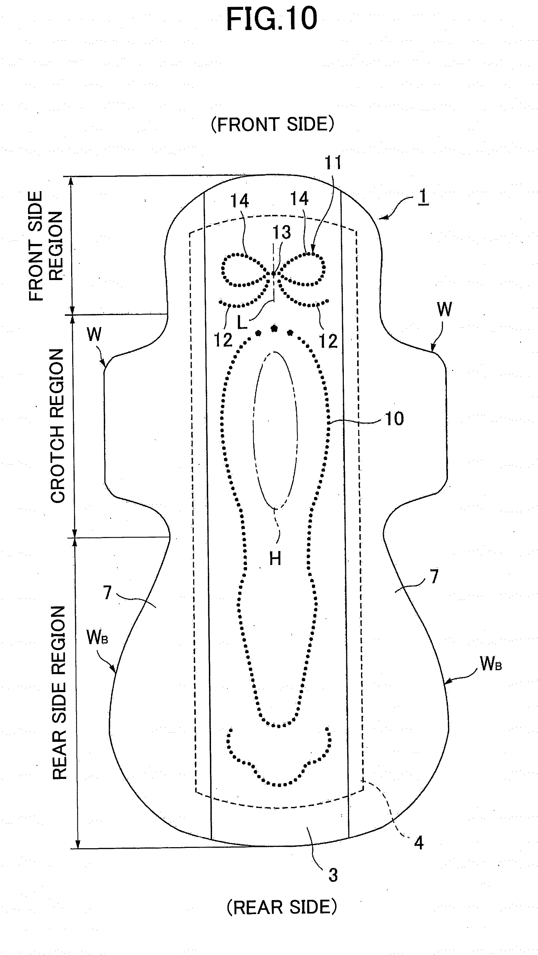

In the above-described embodiment, a so-called daytime sanitary napkin 1, in which a body fluid discharging portion corresponding region H is provided in a substantially central portion of a sanitary napkin 1, is illustrated. As shown in Figs. 10 to 12, a so-called nighttime sanitary napkin 1 is possible. The nighttime sanitary napkin 1 is constituted of a crotch region below the hip including a body fluid discharge portion corresponding portion where the wing-like flaps W and W are provided on both edges, a front side region below the hip up to a front end of a sanitary napkin 1 in which a lower abdomen of a wearer is covered at the time of wearing, and a rear side region below the hip up to a rear end of a sanitary napkin 1 in which a region covers the buttock of a wearer at the time of wearing, wherein the rear side region is formed longer than the front side region and, a pair of left and right hip-hold flaps W_{B} and W_{B} that project outward in the width direction, are formed on the both edges of the rear side region.

In Fig. 10, a single leakproof compressed portion 11 is provided only on the front side of the body fluid discharging portion corresponding region H, that is, in the front side region, but not in the rear side region. By providing the leakproof compressed portion 11 on the front side of the body fluid discharging portion corresponding region H, it is possible to prevent the body fluid from diffusing in the lengthwise direction of the napkin in the front side region that is relatively narrow compared to the rear side region, and leakage from the front end can be prevented.

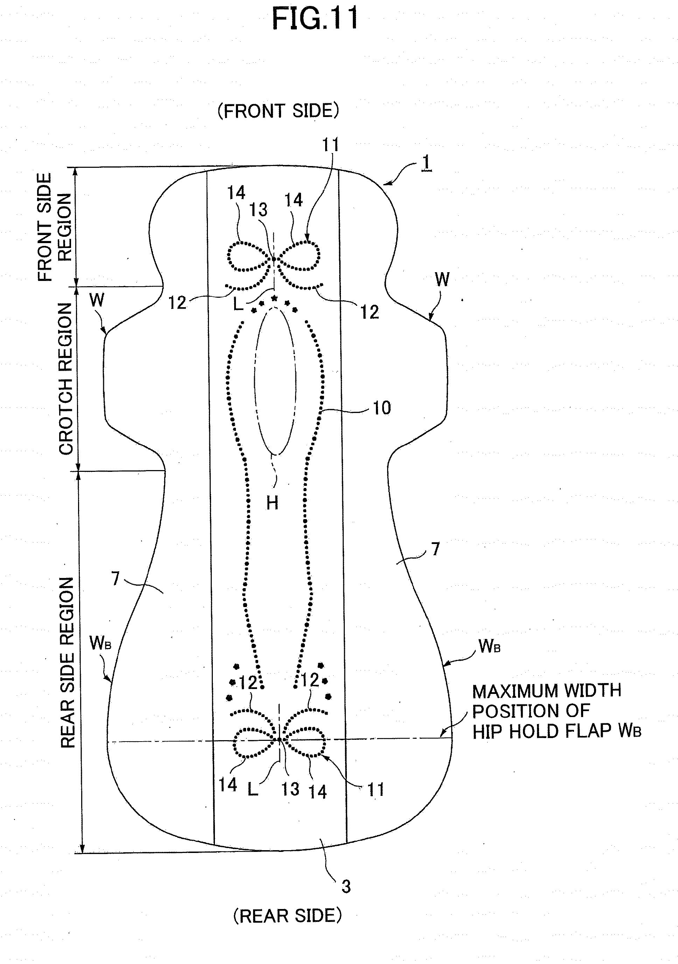

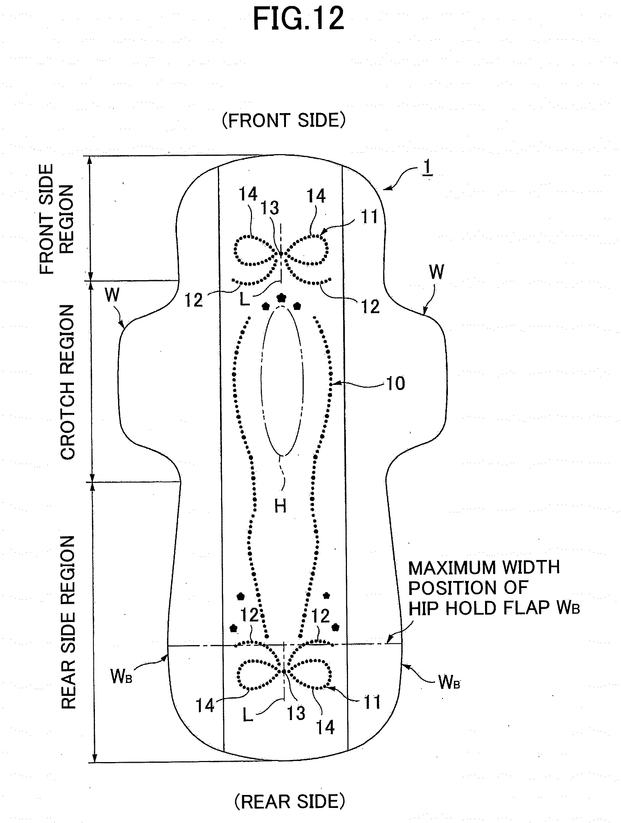

In FIGS. 11 and 12, a single leakproof compressed portion 11 is provided on each of a front side and a rear side of the fluid discharging portion corresponding region H, that is, on the crotch region from below the hips to the front side, and on the rear side. In particular, it is preferable that the leakproof compressed portion 11 provided in the rear side region be disposed so as to overlap the maximum width position of the hip-hold flap W_{B} (the position where the hip-hold flap W_{B} protrudes outwardly in the width direction and the position indicated by the single dotted line in the drawing). Thus, even when part of the body fluid diffusing in the longitudinal direction diffuses in the width direction by the leakproof compressed portion 11, because the hip-hold flap W_{B} extends in the width direction, the leakage from the edge in the width direction can be prevented. When the leakproof compressed portion 11 provided in the rear side region overlaps the maximum width position of the hip-hold flap W_{B}, the leakproof compressed portion 11 may be disposed so that the collection portion 13 of the leakproof compressed portion 11 overlaps the maximum width position as shown in Fig. 11, or the leakproof compressed portion 11 may be disposed so that the liquid induction portion 12 of the leakproof compressed portion 11 overlaps the maximum width position as shown in Fig. 12. In the former case (1), even if the body fluid collected in the collection portion 13 diffuses in the width direction through the liquid diffusion portion 14, the maximum width position of the hip-hold flap W_{B} allows for more reliable prevention of leakage from the edge in the width direction. In the latter case (2), even when the body fluid diffuses outward through the liquid induction portion 12, the maximum width position of the hip-hold flap WB allows for more reliable prevention of leakage from the edge in the width direction.

The leakproof compression portion 11 may be provided only on the rear side of the body fluid discharging portion corresponding portion H, that is, on the rear side of the body fluid discharging portion, and may not be provided in the crotch region below the hips and the front side region. In Figs. 10 to 12, the high compressed portion of the compressed portion composed of the low compressed portion and the high compressed portion is illustrated by a black collapsed portion, and the outer edge of the low compressed portion is not illustrated.

The present application is based upon and claims priority of Japanese Patent Application No. 2018-048764 filed on March 16, 2018, with Japanese Patent Office.

### Description of Reference Symbols

- 1: sanitary napkin
- 2: back sheet
- 3: top sheet
- 4: absorbent body
- 5: encapsulating sheet
- 6: second sheet
- 7: side non-woven fabric
- 10: body fluid discharging compressed portion
- 11: leakproof compressed portion
- 12: liquid induction portion
- 13: liquid collection portion
- 14: liquid diffusion portion
- 15: compressed region
- H: body fluid discharging portion corresponding region
- L: centerline

## Claims

1. A sanitary napkin (1) including a top sheet (3), a back sheet (2), and an absorbent body (4) disposed between the top sheet (3) and the back sheet (2), comprising:
a leakproof compressed portion (11) constituted of a depressed portion formed by depressing at least the absorbent body (4), configured to prevent a body fluid from leaking an end of the absorbent body (4) by changing a diffusion direction of the body fluid diffusing from a region (H) corresponding to a body fluid discharging portion of a wearer, and formed in a predetermined pattern outside the body fluid discharging portion corresponding region (H),
wherein the leakproof compressed portion (11) includes a liquid induction portion (12) that is arranged on both sides of a centerline (L) directed from a proximal side to a distal side, that includes portions whose separation distance on both sides of the centerline (L) decreases toward the distal side from the proximal side, and that is configured to introduce the body fluid diffusing outward from the body fluid discharging portion corresponding region, a body fluid collection portion (13) located on the centerline (L) and more distant from the body fluid discharging portion corresponding region than the liquid induction portion (12) and configured to collect the body fluid introduced by the liquid induction portion (12), and a liquid diffusion portion (14) configured to diffuse the collected liquid onto both sides of the centerline (L),
wherein a separation distance between the leakproof compressed portion (11) and an edge of the absorbent body (4) is not less than 10 mm,
wherein the liquid induction portions (12) on both sides of the centerline (L) has an approximately inverted V shape without its bonded apex as seen from the proximal side to the distal side,
wherein a separation width between ends of the liquid induction portions (12) on the centerline (L) side is from 2 mm to 10 mm, and
wherein a length of the portions whose separation distance on both sides of the centerline (L) decreases toward the distal side from the proximal side is not less than 1/2 relative to the entire length of the liquid induction portion (12) in a direction perpendicular to the centerline (L).

2. The sanitary napkin (1) according to claim 1, wherein the leakproof compressed portion (11) is disposed at least on one of the front side and the rear sides or on both sides of the body fluid discharging portion corresponding region.

3. The sanitary napkin (1) according to claim 1, wherein the liquid induction portions (12) are spaced apart from each other on both sides of the centerline (L) .

4. The sanitary napkin (1) according to claim 1, wherein the liquid induction portion (12) and the liquid collection portion (13) are arranged consecutively or spaced apart from each other.

5. The sanitary napkin (1) according to claim 1, wherein the liquid induction portion (12) is formed so as to gradually increase an average density of fibers toward the centerline (L).

6. The sanitary napkin (1) according to claim 1, wherein the liquid collection portion (13) has a low compressed portion and a high compressed portion, and wherein the high compressed portion is formed into a circular shape, an X shape, or a diamond shape in a plan view.

7. The sanitary napkin (1) according to claim 1, wherein the liquid diffusion portion (14) extends from the liquid collection portion (13) on both sides of the centerline (L) in a different direction.

8. The sanitary napkin (1) according to claim 7, wherein at least two of the plurality of liquid diffusion portions (14) extending across the centerline (L) are connected at a tip thereof.

9. The sanitary napkin (1) according to claim 1, wherein the leakproof compressed portion (11) is formed in a right-left symmetrical or right-left asymmetric relation to the centerline (L).

## Patentansprüche

1. Hygienebinde (1) mit einer oberen Lage (3), einer rückwärtigen Lage (2) und einem absorbierenden Körper (4), der zwischen der oberen Lage (3) und der rückwärtigen Lage (2) vorgesehen ist, aufweisend:
einen auslaufsicheren komprimierten Abschnitt (11), der aus einem zusammengedrückten Abschnitt besteht, welcher durch Zusammendrücken mindestens des absorbierenden Körpers (4) gebildet ist, der dafür ausgelegt ist, den Austritt einer Körperflüssigkeit an einem Ende des absorbierenden Körpers (4) zu verhindern, indem eine Verteilungsrichtung der Körperflüssigkeit geändert wird, die sich ausgehend von einem Bereich (H) verteilt, der einem Körperflüssigkeits-Ausleitabschnitt eines Trägers entspricht, und in einer vorbestimmten Struktur außerhalb des dem Körperflüssigkeits-Ausleitabschnitt entsprechenden Bereichs (H) ausgebildet ist,
wobei der auslaufsichere komprimierte Abschnitt (11) einen Flüssigkeitseinleitabschnitt (12), der auf beiden Seiten einer Mittellinie (L) angeordnet ist, von einer proximalen Seite zu einer distalen Seite gerichtet, der Abschnitte umfasst, deren Trennabstand auf beiden Seiten der Mittellinie (L) von der proximalen Seite zur distalen Seite hin abnimmt, und der dafür ausgelegt ist, die sich von dem dem Körperflüssigkeits-Ausleitabschnitt entsprechenden Bereich nach außen hin verteilende Körperflüssigkeit einzuleiten, einen Körperflüssigkeits-Sammelabschnitt (13), der sich auf der Mittellinie (L) befindet und von dem dem Körperflüssigkeits-Ausleitabschnitt entsprechenden Bereich weiter entfernt ist als der Flüssigkeitseinleitabschnitt (12) und dafür ausgelegt ist, die durch den Flüssigkeitseinleitabschnitt (12) eingeleitete Körperflüssigkeit zu sammeln, und einen Flüssigkeitsverteilungsabschnitt (14) umfasst, der dafür ausgelegt ist, die gesammelte Flüssigkeit auf beiden Seiten der Mittellinie (L) zu verteilen,
wobei ein Trennabstand zwischen dem auslaufsicheren komprimierten Abschnitt (11) und einem Rand des absorbierenden Körpers (4) nicht unter 10 mm beträgt,
wobei die Flüssigkeitseinleitabschnitte (12) auf beiden Seiten der Mittellinie (L) ohne ihren verbundenen Scheitelpunkt eine annähernd umgekehrte V-Form haben, von der proximalen Seite zur distalen Seite betrachtet,
wobei eine Trennbreite zwischen Enden der Flüssigkeitseinleitabschnitte (12) aufseiten der Mittellinie (L) 2 mm bis 10 mm beträgt, und
wobei eine Länge der Abschnitte, deren Trennabstand auf beiden Seiten der Mittellinie (L) von der proximalen Seite zur distalen Seite hin abnimmt, in einer zur Mittellinie (L) senkrechten Richtung nicht weniger als die Hälfte relativ zur Gesamtlänge des Flüssigkeitseinleitabschnitts (12) beträgt.

2. Hygienebinde (1) nach Anspruch 1, wobei der auslaufsichere komprimierte Abschnitt (11) an der Vorderseite oder an der Rückseite oder an beiden Seiten des dem Körperflüssigkeits-Ausleitabschnitt entsprechenden Bereichs vorgesehen ist.

3. Hygienebinde (1) nach Anspruch 1, wobei die Flüssigkeitseinleitabschnitte (12) auf beiden Seiten der Mittellinie (L) voneinander beabstandet sind.

4. Hygienebinde (1) nach Anspruch 1, wobei der Flüssigkeitseinleitabschnitt (12) und der Flüssigkeitssammelabschnitt (13) hintereinander oder voneinander beabstandet angeordnet sind.

5. Hygienebinde (1) nach Anspruch 1, wobei der Flüssigkeitseinleitabschnitt (12) so ausgebildet ist, dass die durchschnittliche Faserdichte zur Mittellinie (L) hin allmählich zunimmt.

6. Hygienebinde (1) nach Anspruch 1, wobei der Flüssigkeitssammelabschnitt (13) einen schwach komprimierten Abschnitt und einen stark komprimierten Abschnitt hat, und wobei der stark komprimierte Abschnitt in der Draufsicht in einer Kreisform, einer X-Form oder einer Rautenform ausgebildet ist.

7. Hygienebinde (1) nach Anspruch 1, wobei sich der Flüssigkeitsverteilungsabschnitt (14) ausgehend vom Flüssigkeitssammelabschnitt (13) auf beiden Seiten der Mittellinie (L) in einer jeweils anderen Richtung erstreckt.

8. Hygienebinde (1) nach Anspruch 7, wobei mindestens zwei aus der Vielzahl von Flüssigkeitsverteilungsabschnitten (14), die sich über die Mittellinie (L) hinweg erstrecken, an deren Spitze verbunden sind.

9. Hygienebinde (1) nach Anspruch 1, wobei der auslaufsichere komprimierte Abschnitt (11) in einer rechts-links-symmetrischen oder einer rechts-links-asymmetrischen Beziehung zur Mittellinie (L) ausgebildet ist.

## Revendications

1. Serviette hygiénique (1) comprenant une feuille supérieure (3), une feuille arrière (2) et un corps absorbant (4) prévu entre la feuille supérieure (3) et la feuille arrière (2), comprenant :
une section comprimée anti-fuite (11) constituée d'une section comprimée formée en comprimant au moins le corps absorbant (4), qui est adaptée pour empêcher la fuite d'un liquide corporel à une extrémité du corps absorbant (4) en changeant une direction de distribution du liquide corporel qui se distribue à partir d'une zone (H) correspondant à une section de décharge de liquide corporel d'un porteur et est formée dans une structure prédéterminée à l'extérieur de la zone (H) correspondant à la section de décharge de liquide corporel,
sachant que la section comprimée anti-fuite (11) comprend une section d'introduction de liquide (12) disposée de part et d'autre d'une ligne centrale (L), dirigée d'un côté proximal vers un côté distal, qui comprend des sections dont la distance de séparation diminue de part et d'autre de la ligne centrale (L) du côté proximal vers le côté distal, et qui est adaptée pour introduire le liquide corporel se distribuant vers l'extérieur depuis la zone correspondant à la section de décharge de liquide corporel, une section de collecte de liquide corporel (13) située sur la ligne centrale (L) et plus éloignée de la zone correspondant à la section de décharge de liquide corporel que la section d'introduction de liquide (12) et adaptée pour collecter le liquide corporel introduit par la section d'introduction de liquide (12), et une section de distribution de liquide (14) adaptée pour distribuer le liquide collecté des deux côtés de la ligne centrale (L),
sachant qu'une distance de séparation entre la section comprimée anti-fuite (11) et un bord du corps absorbant (4) n'est pas inférieure à 10 mm,
sachant que les sections d'introduction de liquide (12) des deux côtés de la ligne centrale (L), sans leur sommet relié, ont une forme approximativement en V inversé, vu du côté proximal vers le côté distal,
sachant qu'une largeur de séparation entre des extrémités des sections d'introduction de liquide (12) sur le côté de la ligne centrale (L) est de 2 mm à 10 mm, et
sachant qu'une longueur des sections dont la distance de séparation diminue des deux côtés de la ligne centrale (L) depuis le côté proximal vers le côté distal, dans une direction perpendiculaire à la ligne centrale (L), n'est pas inférieure à la moitié par rapport à la longueur totale de la section d'introduction de liquide (12).

2. La serviette hygiénique (1) selon la revendication 1, sachant que la section comprimée anti-fuite (11) est prévue sur le côté avant ou sur le côté arrière ou sur les deux côtés de la zone correspondant à la section de décharge de liquide corporel.

3. La serviette hygiénique (1) selon la revendication 1, sachant que les sections d'introduction de liquide (12) sont espacées les unes des autres de part et d'autre de la ligne centrale (L).

4. La serviette hygiénique (1) selon la revendication 1, sachant que la section d'introduction de liquide (12) et la section de collecte de liquide (13) sont disposées l'une derrière l'autre ou sont espacées l'une de l'autre.

5. La serviette hygiénique (1) selon la revendication 1, sachant que la section d'introduction de liquide (12) est formée de telle sorte que la densité moyenne des fibres augmente progressivement vers la ligne centrale (L).

6. La serviette hygiénique (1) selon la revendication 1, sachant que la section de collecte de liquide (13) a une section faiblement comprimée et une section fortement comprimée, et sachant que la section fortement comprimée a une forme circulaire, une forme en X ou une forme de losange en vue en plan.

7. La serviette hygiénique (1) selon la revendication 1, sachant que la section de distribution de liquide (14) s'étend dans une direction différente de chaque côté de la ligne centrale (L) à partir de la section de collecte de liquide (13).

8. La serviette hygiénique (1) selon la revendication 7, sachant qu'au moins deux de la pluralité de sections de distribution de liquide (14) s'étendant à travers la ligne centrale (L) sont reliées à son extrémité.

9. La serviette hygiénique (1) selon la revendication 1, sachant que la section comprimée anti-fuite (11) est formée selon une relation symétrique droite-gauche ou asymétrique droite-gauche par rapport à la ligne centrale (L).
